# EUROPEAN PATENT APPLICATION

(11) **EP 4 289 413 A1**
(43) Date of publication of application: **13.12.2023**
(21) Application number: 23175997.8
(22) Date of filing: 30.05.2023
(51) Int. Cl.: A61K 8/9789, A61Q 19/08, A23L 33/105

(54) **USE OF CRASSOCEPHALUM RABENS EXTRACT FOR IMPROVING SKIN CONDITION**

(30) Priority: 06.06.2022 US 202263349299 P
(71) Applicant: Greenyn Biotechnology Co., Ltd, Taichung City (TW)
(72) Inventor: Hsu, Pang-Kuei, Taichung City (TW); Lin, Yu Cheng, Taichung City (TW); Chuang, Wei-Hsiu, Taichung City (TW); Kuan, Chen-Meng, Taichung City (TW); Wu, Chia-Feng, Taichung City (TW)
(74) Representative: Lermer, Christoph

(57) **Abstract**

Use of a *Crassocephalum rabens* extract in improving skin condition is provided. Since the *Crassocephalum rabens* extract has activity in enhancing expression of collagen genes and/or of elastin genes, by administrating an effective amount of the *Crassocephalum rabens* extract or a composition containing the effective amount of the *Crassocephalum rabens* extract to an individual, levels of collagen and elastin in skin of the individual can be effectively increased, so as to improve skin texture and retard skin aging.

## Description

### BACKGROUND OF THE INVENTION

### 1. Technical Field

The present invention relates to novel use of a plant extract, and more particularly to use of Crassocephalum rabens extract in improving skin condition.

### 2. Description of Related Art

Modern people have been paying more and more attention to individual appearances, in particular skin condition. There are many factors having impact on skin texture, such as dietary habit, living habit, exercise habit, environmental factors, pollution exposure, and age, in additional genetic factors. For example, aging can lead to decreased collagen and reduced elastic fibers, making skin look dull and less supple and bringing about wrinkles. Ultraviolet (UV) rays increase free radicals in our bodies and fuel the emersion of spots and wrinkles.

*Crassocephalum rabens,* also known as Asteraceae, is a herbaceous species of the family Asteraceae. As recited in Taiwan Herbal Pharmacopeia, *Crassocephalum rabens* has the efficacy of clearing heat, detoxifying, smoothening qi, and diuresis, and is known to be effective in treating hypertension, cephalalgia, and insect bites. According to recent studies, *Crassocephalum rabens* has active ingredients remedial to cancers and inhibitive to inflammatory responses, making it useful in developing anti-cancer drugs and anti-inflammatory drugs. Also suggested in other studies, dLGG (1,2-di-O-linolenoyl-3-O-β-galactopyranosyl-sn-glycerol), the main active ingredient of *Crassocephalum rabens* helps reduce recurrence of breast cancer and restrain pulmonary metastasis.

### BRIEF SUMMARY OF THE INVENTION

One primary objective of the present invention is to provide use of a *Crassocephalum rabens* extract in improving skin condition, wherein administration of an effective amount of the extract to an individual elevates levels of collagen and elastin in skin cells of the individual, thereby maintaining skin plumpness, reducing formation of wrinkles, and smoothening formed skin lines.

Another primary objective of the present invention is to provide use of a *Crassocephalum rabens* extract in improving skin condition, wherein in virtue of activity of the extract in inhibiting hyaluronidase, preventing formation of inflammatory factors and reactive oxygen species (ROS), and reducing oxidative stress, administration of an effective amount of the extract to an individual improves hydration in skin cells, mitigates formed skin spots, and make sensitive skin healthy.

Hence, in order to achieve the foregoing objectives, the present invention provides use of a composition containing a *Crassocephalum rabens* extract in improving skin texture or in caring skin. Specifically, the composition containing the *Crassocephalum rabens* extract is used to lighten skin spots, increase collagen in skin cells, and decrease skin pores in number and/or shrink skin pores in size.

In one embodiment of the present invention, the *Crassocephalum rabens* extract is prepared from *Crassocephalum rabens* through an extraction process, and the *Crassocephalum rabens* extract contains a predetermined proportion of dLGG (1,2-di-O-linolenoyl-3-O-β-galactopyranosyl-sn-glycerol). Therein, the extraction process is well known in the art and thus will not be described in detail herein.

In a further embodiment of the present invention, the *Crassocephalum rabens* extract contains 200-250 mg/mL of dLGG.

In one embodiment of the present invention, the composition containing the *Crassocephalum rabens* extract is a nutritional supplement, a food, or a skincare product for external use.

Another embodiment of the present invention provides use of the *Crassocephalum rabens* extract in preparing an anti-wrinkle composition. In particular, since the *Crassocephalum rabens* extract of the present invention has activity of enhancing gene expression in skin related to collagen and/or elastin, by administrating the anti-wrinkle composition to an individual, the content of collagen and/or elastin in the skin of the individual can be effectively increased, thereby preventing formation of wrinkles and smoothening formed wrinkles.

### BRIEF DESCRIPTION OF THE SEVERAL VIEWS OF THE DRAWINGS

FIG. 1A is an HPLC spectrum of a *Crassocephalum rabens* extract of the present invention made using supercritical extraction.
FIG. 1B is an HPLC spectrum of a *Crassocephalum rabens* extract of the present invention made using alcoholic extraction.
FIG. 1C is an HPLC spectrum of a *Crassocephalum rabens* extract of the present invention made using aqueous extraction.
FIG. 2 shows results of HPLC analysis of the *Crassocephalum rabens* extract of the present invention, wherein the HPLC spectrum of the disclosed extract is at the top and the HPLC spectrum of a dLGG standard is at the bottom.
FIG. 3 are images for quantitative comparison of face pores of a user obtained by means of the VISIA Complexion Analysis System before and after her use of the inventive extract.
FIG. 4A shows quantitative variations of face pores of the user over time related to her use of the inventive extract.
FIG. 4B shows qualitive variations of face textures of the user over time related to her use of the inventive extract.
FIG. 5 are images for comparison of face spots of a user obtained by means of the VISIA Complexion Analysis System before and after her use of the inventive extract.
FIG. 6A shows variations of UV spots of the user over time related to her use of the inventive extract.
FIG. 6B shows variations of spots other than UV spots of the user over time related to her use of the inventive extract.
FIG. 7 shows variations of skin wrinkles of users after their use of the inventive extract, wherein * indicates a p value <0.05 between two groups.
FIG. 8 are images of different subjects for comparison of variations of wrinkles obtained by means of the VISIA Complexion Analysis System before and after their oral administration of the inventive extract.
FIG. 9 shows results of analysis of skin collagen levels of users before and after their use of the inventive extract, wherein *** indicates a p value <0.001 between two groups.
FIG. 10 are images showing collagen density in different subjects obtained before and after their oral administration of the inventive extract.
FIG. 11 shows variations of skin glow of users after their use of the inventive extract, wherein *** indicates a p value <0.001 between two groups.
FIG. 12 shows variations of UV spots of users after their use of the inventive extract, wherein ** indicates a p value <0.01 between two groups.
FIG. 13A shows gene expressions of COL1A1, COL1A2, ELN, etc. in cells of users from different groups.
FIG. 13B shows the statistical results of quotative analysis of data shown in FIG. 12A.

### DETAILED DESCRIPTION OF THE INVENTION

The present invention discloses use of *Crassocephalum rabens* extract in improving skin condition. Specifically, since the *Crassocephalum rabens* extract of the present invention has activity in enhancing expression of collagen genes and/or of elastin genes, by administrating an effective amount of the *Crassocephalum rabens* extract or a composition containing the effective amount of the *Crassocephalum rabens* extract to an individual, levels of collagen and elastin in skin of the individual can be effectively increased, so as to improve skin texture and retard skin aging.

As used herein, phrases "improving skin texture" and "improving skin condition" refer to quantitively reducing wrinkles, smoothening formed lines, improving skin color, inhibiting formation of spots, reducing pores in both size and number, lightening formed pigmentation or spots, improving uneven skin tone, improving skin redness, elevating collagen abundance, enhancing skin plumpness, increase skin glow, improving skin brightness, etc.

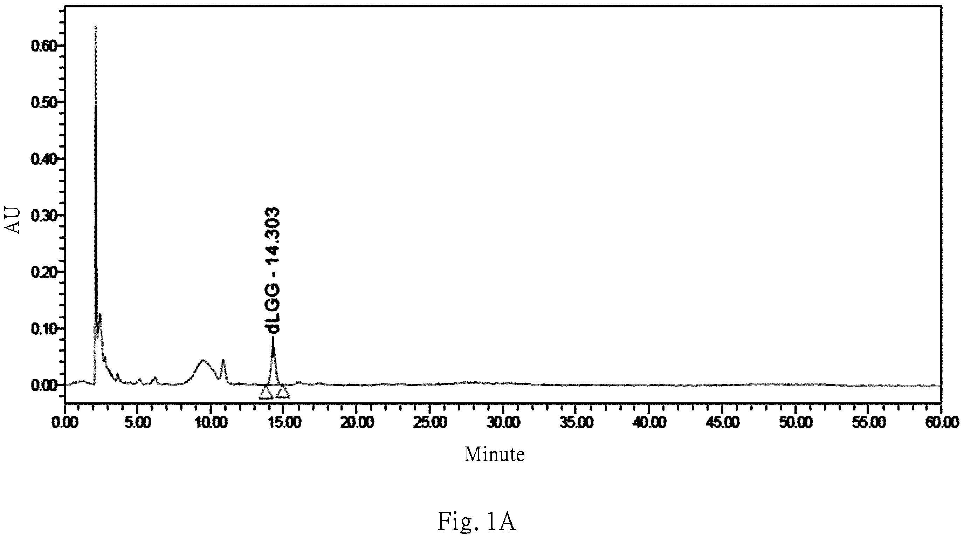

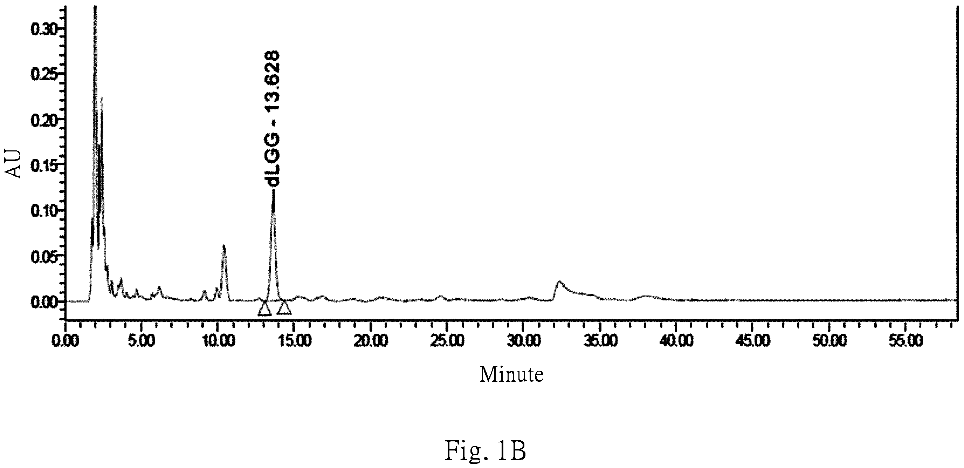

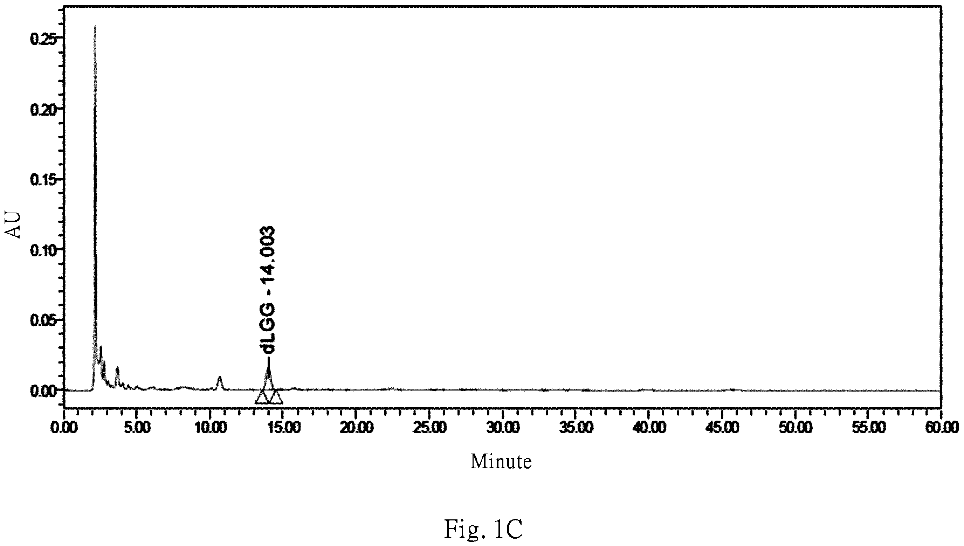

The *Crassocephalum rabens* extract of the present invention is a mixture rich in dLGG (1,2-di-O-linolenoyl-3-O-β-galactopyranosyl-sn-glycerol) obtained by processing a *Crassocephalum rabens* material with an extraction process. As shown in FIG. 1A through FIG. 1C, the *Crassocephalum rabens* extract of the present invention may be produced using different extraction processes, such as supercritical extraction, alcoholic extraction, aqueous extraction, or any combination thereof, without limitation, as long as the obtain *Crassocephalum rabens* extract contains sufficient dLGG (1,2-di-O-linolenoyl-3-O-β-galactopyranosyl-sn-glycerol) as its major marker constituent.

As used herein, the term "*Crassocephalum rabens* material" refers to a fresh plant of *Crassocephalum rabens* or a powder processed from *Crassocephalum rabens.*

As used herein, the term "extraction processes" refers to methods for isolating or obtaining a specific component from a mixture using different solvents, and may be aqueous extraction, alcoholic extraction, supercritical extraction, or any combination thereof. Therein, for aqueous extraction, an exemplificative extraction solvent is water; for alcoholic extraction, an exemplificative extraction solvent is ethyl alcohol having a concentration equal to or greater than 95%; and for supercritical extraction, exemplificative extraction solvents are carbon dioxide and ethyl alcohol having a concentration equal to or greater than 95%.

As used herein, the term "composition" refers to a product capable of improving or maintaining skin texture. The composition is not limited in terms of administration, and thus may be administrated externally or orally. The composition is not limited in terms of formulation, and thus may be in the form of emulsion, paste, liquid, tablets, powder, or capsules. In some examples, the disclosed composition may be a skincare product for external use, a nutritional supplement, a food product, etc.

As used herein, the term "effective amount" refers to a dosage capable of achieving the intended effects of the object it related to, and such a dosage can vary depending on parameters such as the selected formulation, the selected delivery route, and the individual administrated. For example, to administrate an adult subject with the *Crassocephalum rabens* extract of the present invention through orally for improving his/her skin texture in 14 days or more after start of the administration, the effective amount of the *Crassocephalum rabens* extract preferably ranges between 100 and 200mg/day, and more preferably is of 180mg/day. Alternatively, in the same setting except that the inventive extract is administrated through external use, the effective amount of the *Crassocephalum rabens* extract is preferably of 0.66mg/day or more, and more preferably ranges between 0.66 and 3.3 mg/day. Therein, the preferred dosage for external use can be determined through calculation based on dosage proven to be effective in *In vitro* tests, such as 10-50 ug/mL.

As used herein, the term "administration" or "administrating" refers to the way by which the *Crassocephalum rabens* extract of the present invention or a composition containing the extract is delivered to a subject, and may include oral medication and external use, which further includes, but is not limited to, applying and spraying the extract or the composition onto skin.

As used herein, the term "skin spots" refers to visible marks on skin, including sunburns, stains, chloasma, sun spots, age spots, scars, and other consequences of pigmentation.

As used herein, the term "UV spots" refers to spots related to increased melanin caused by exposure to ultraviolet rays. Generally, UV spots are black or close to black.

As used herein, the term "brown spots" refers to pigmentation formed in or under the epidermis. Generally, brown spots are brown or tan, such as freckles.

As used herein, the terms "about" and "approximately" are used to describe and account for small variations as results of inaccuracy associated with a value that are tolerable to a person of ordinary skill in the art, and the variations may vary with methods by which the value was measured or calculated but usually do not exceed a range equal to 10 % of the value 10%.

For further illustrating the technical features and effects of the present invention, the following description, in conjunction with the accompanying drawings and preferred embodiments, is set forth as below.

All human trials in examples provided hereunder were performed to conform to all applicable rules. Since cells used in *in vitro* trials of the present invention are all commercially available, the requirements for deposit are not applicable.

It is to be noted that data disclosed in Table 3 through Table 5 related to the examples described below were obtained through analysis of images captured using a VISIA Complexion Analysis System (Canfield Imaging Systems, Fairfield, NJ) and therefore no unit is applied to these values.

### Example 1: Preparation of the Crassocephalum rabens extract

Powder fresh *Crassocephalum rabens* was prepared and then treated with extraction, filtration, centrifugation, hydration, and lyophilization as known in the art to yield a *Crassocephalum rabens* extract. Therein, the extraction process was performed with 95% ethyl alcohol as the extraction solvent. The *Crassocephalum rabens* extract was found rich in dLGG (1,2-di-O-linolenoyl-3-O-β-galactopyranosyl-sn-glycerol), and used in examples described below.

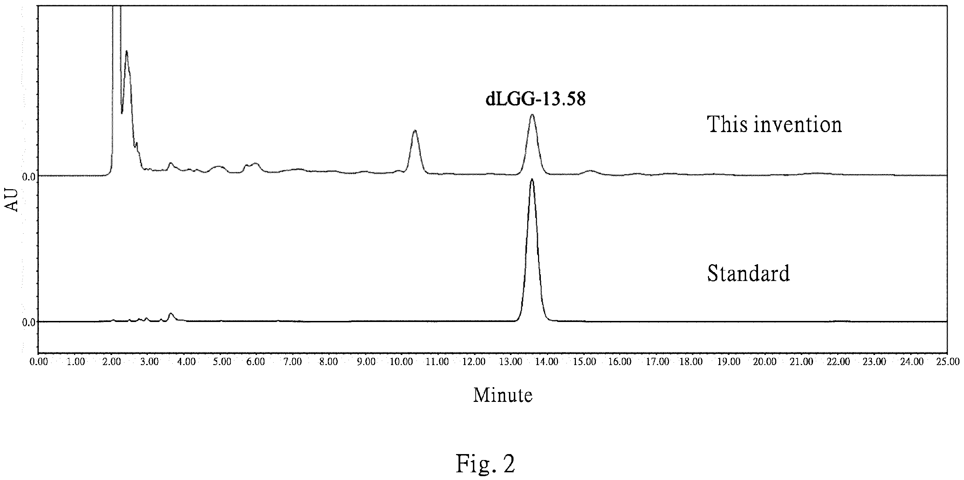

FIG. 2 shows results of HPLC analysis of the *Crassocephalum rabens* extract of the present invention. The HPLC process was performed under conditions of: 98% methanol as the solvent; the flow rate of 1.0mL/min; gradient elution; the injection volume of 10µL; the retention time of 60 minutes; the column temperature at 30°C; and detection at 210nm. According to the results shown in FIG. 2, dLGG was detected in the *Crassocephalum rabens* extract of the present invention at the retention time of 13.05 ± 0.80 minutes, with the concentration of 218.05 mg/mL.

### Example 2: Evaluation of efficacy of skin improvement (I)

Totally 12 subjects were assembled and administrated with the *Crassocephalum rabens* extract in the formulation of capsules each containing 180mg of the extract. The dosage was one capsule per day. The experiment lasted for 4 weeks. In Weeks 0, 2, and 4, the subjects were evaluated for skin condition and their self-comparison for skin condition before and after administration of the capsules. The evaluation was perform using a VISIA Complexion Analysis System.

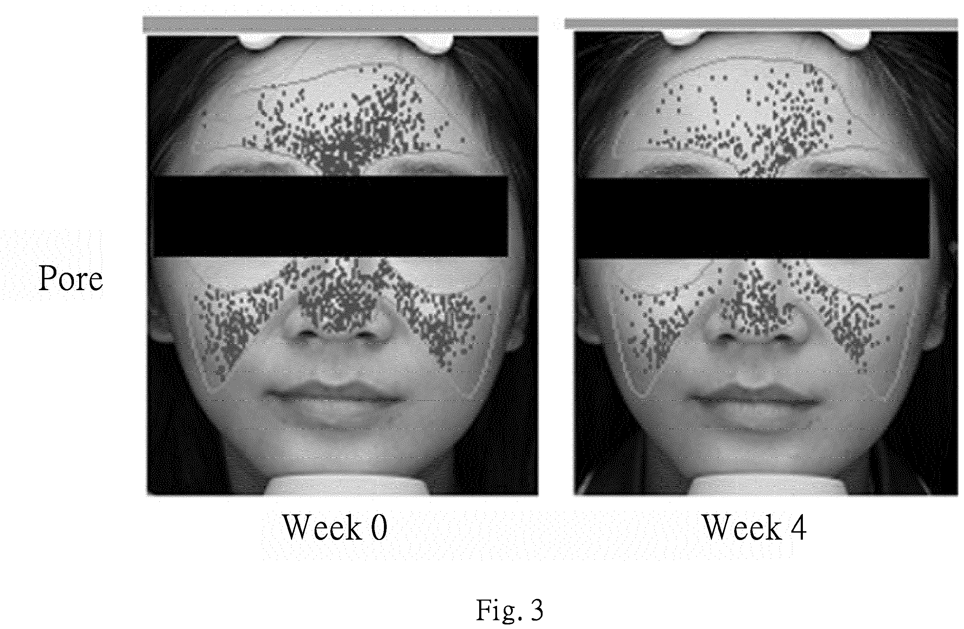

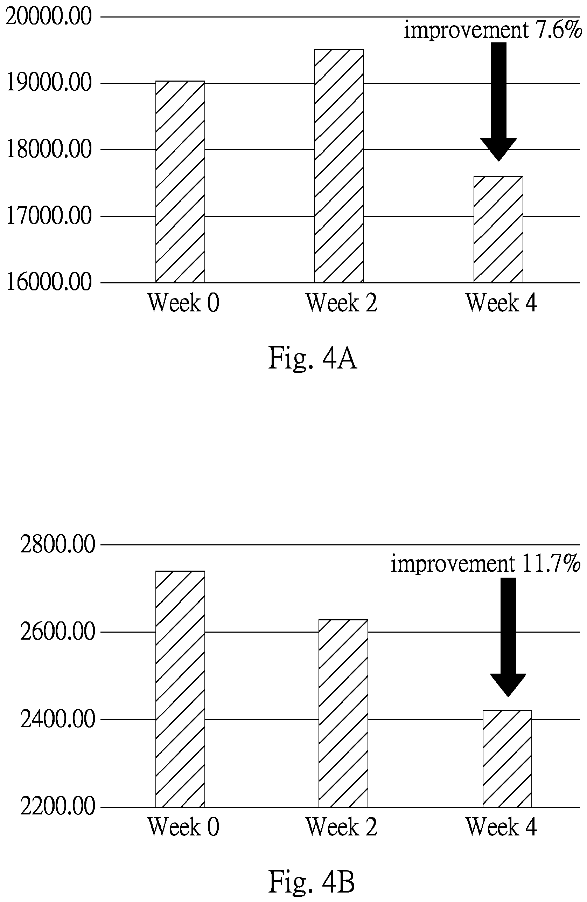

The results shown in FIG. 3 and FIG. 4 demonstrate that nearly 92% of the subjects had improvement in both skin pore and skin texture by 7-11%, respectively. Therein, the subject reporting maximum improvement had a reduction of skin pores of about 49%.

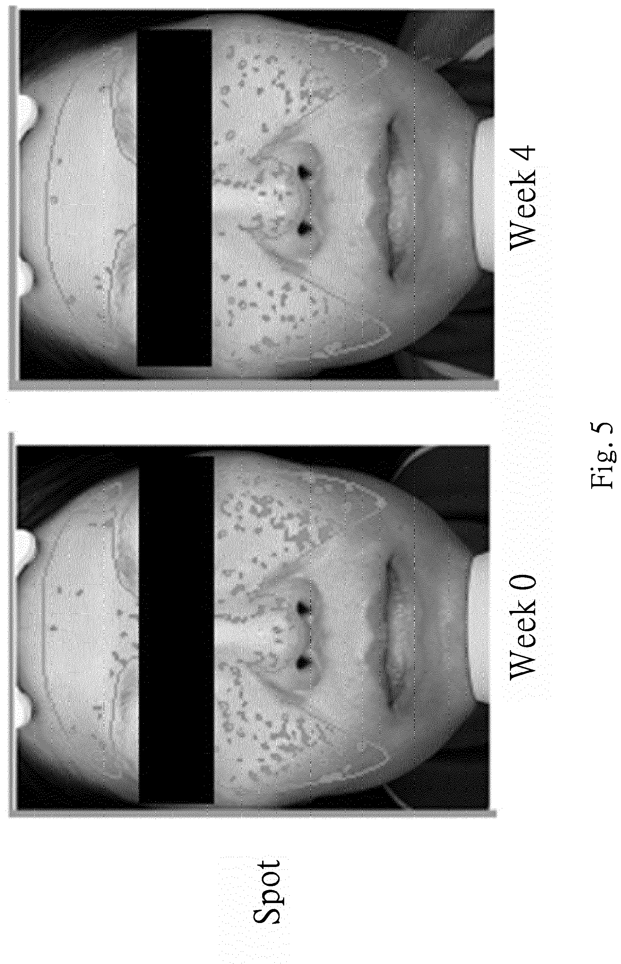

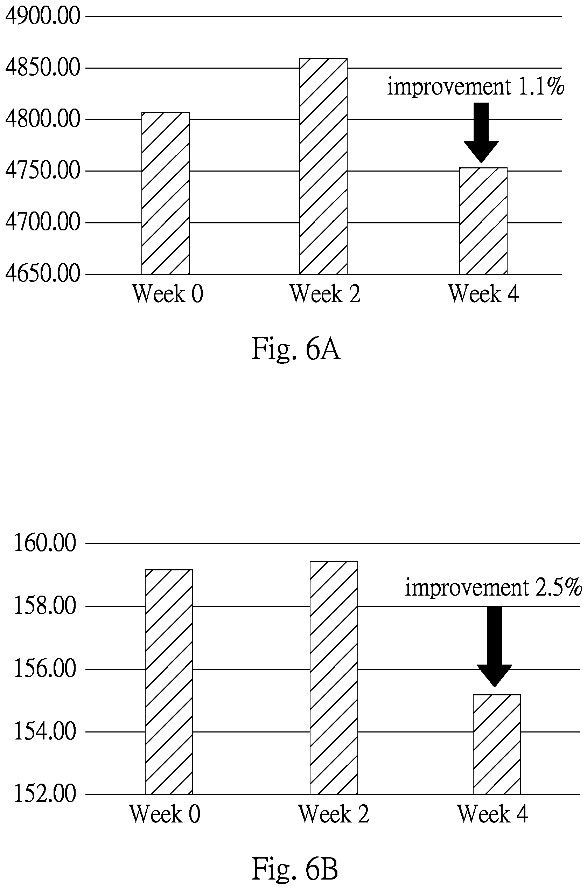

As shown by the data in FIG. 5 and FIG. 6, nearly 70% of the subjects had improvement in skin spots, suggesting that the *Crassocephalum rabens* extract of the present invention helped prevent melanin pigmentation and formation of spots. Therein, the subject reporting maximum improvement had a reduction of spots and pores of about 28%.

### Example 3: Evaluation of efficacy of skin improvement (II)

Totally 20 subjects were assembled and randomly divided into two groups for double-blind study. One of the groups was the experimental group, administrated with the *Crassocephalum rabens* extract in the formulation of capsules each containing 180mg of the extract at the dosage of one capsule per day. The other group is the placebo group. The experiment lasted for 4 weeks. In Weeks 0, 2, and 4, the subjects were evaluated for skin condition in terms of skin glow, moisture, elasticity, fleck, UV spot, brown spot, red area, wrinkle, texture, pore, porphyrin, and collagen density. The results are shown in FIG. 5 through FIG. 10.

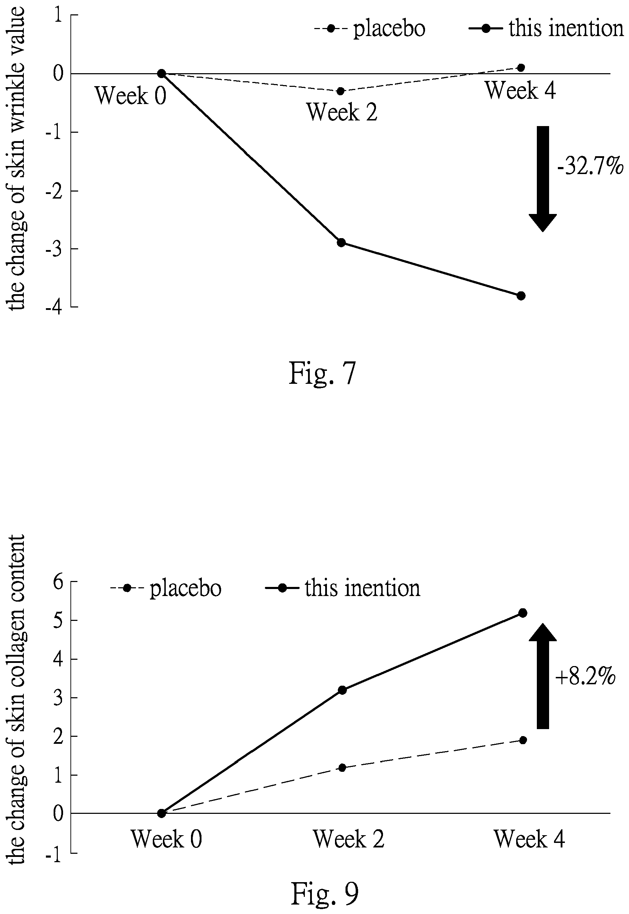

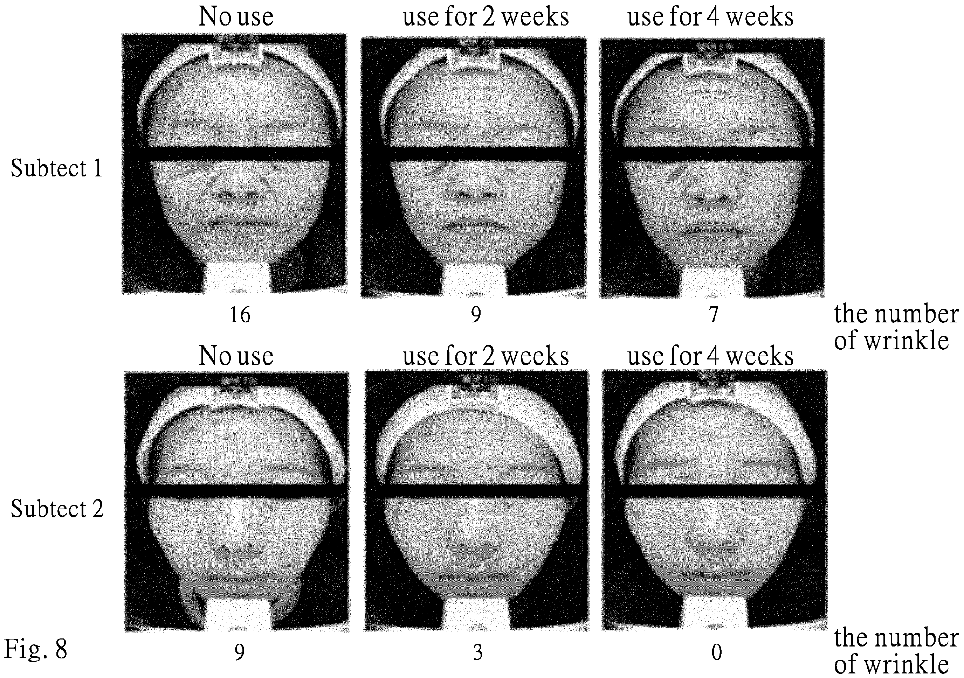

According to the results shown in FIG. 7 and FIG. 8, the subjects in the experimental group had improvement in wrinkle of more than 50%, and has improvement of 32.7% as compared to the placebo group, which indicates that the *Crassocephalum rabens* extract of the present invention showed effectiveness in reducing the number of wrinkles.

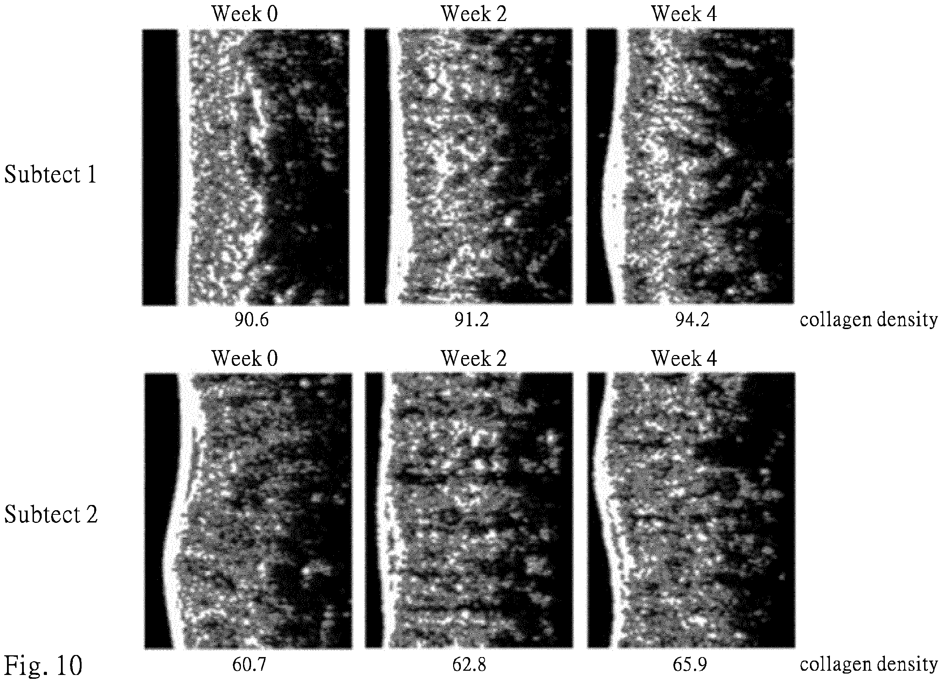

The data shown in FIG. 9 and FIG. 10 disclose that after using the inventive extract, the subjects of the experimental group saw increase in the level of skin collagen of 8.2% as compared to the placebo group. This result suggests that the *Crassocephalum rabens* extract of the present invention was effective in increasing collagen in skin cells and maintaining skin elasticity and glow.

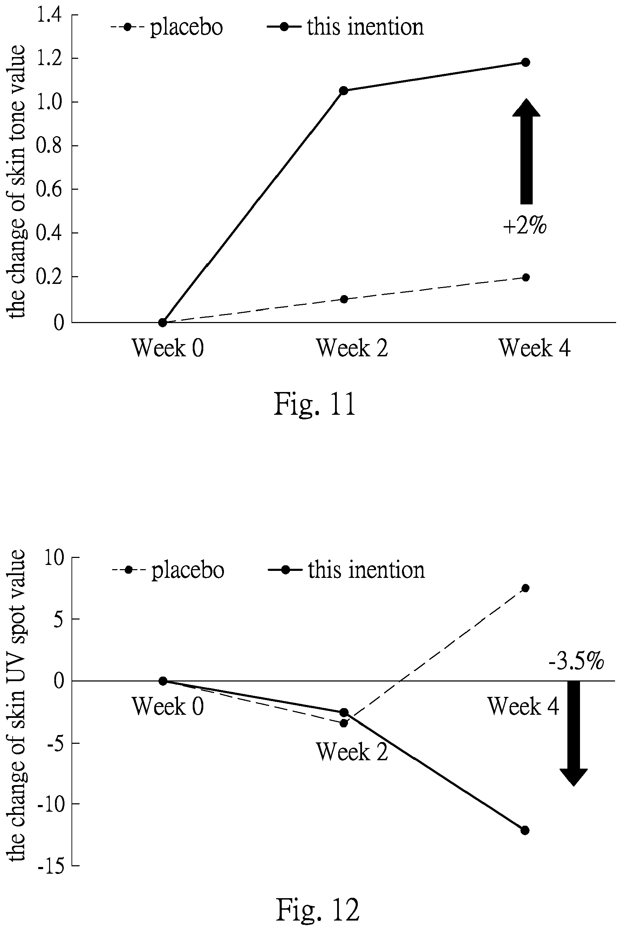

From the data reflected in FIG. 11 and FIG. 12, it is clear that the subjects of the experimental group had more improvement in skin tone than those of the placebo group. In particular, the reduction of UV-incurred spots demonstrates that the *Crassocephalum rabens* extract of the present invention effectively improved skin glow, evened skin tone, and inhibited pigmentation.

### Example 4: Comparison of different functional components

In addition to the *Crassocephalum rabens* extract of the present invention, some other commercially available materials were collected, including oral hyaluronic acid (for continuous use of 20 days), ceramide, and collagen (for continuous use of 6 weeks), to compare their effects in maintaining skin hydrated, reducing wrinkles, and increasing collagen. The results are shown in Table 1 below.

**Table 1: Comparison of different components for skin-improving efficacy**

| Component | Moisture (%) | Wrinkle (%) | Collagen Density (%) |
|---|---|---|---|
| *Crassocephalum rabens* extract | +1.6 | -32.7 | +8.2 |
| Oral hyaluronic acid (for 20 days) | +21.5 | -12.5 | No data |
| Ceramide | +2.7 | -5.8 | No data |
| Collagen (for 6 weeks) | +25.5 | No significant difference | No data |

As shown in Table 1, as compared to the commercially available components tested, the *Crassocephalum rabens* extract of the present invention was more effective in reducing formation of skin lines, smoothening formed wrinkles, and exciting formation of collagen, leading to the increased collagen density, improved skin texture, and less aging impact.

### Example 5: Evaluation of efficacy of skin improvement (III)

For this example, 40 subjects were recruited and randomly grouped into the placebo group or the treatment group, as shown in Table 2. The two groups had the same male-to-female ratio. The placebo group and the treatment group had average ages of 46.4 y/o and 43.9 y/o, respectively. No statistical difference found between the average ages of the two groups. The experiment lasted for 4 weeks, during which period subjects of the treatment group were administrated with capsules each containing 180mg of the *Crassocephalum rabens* extract at dosage of one capsule per day.

**Table 2: Demographic data of subjects**

| | Placebo Group (n = 20) | Treatment Group (n = 20) | P Value |
|---|---|---|---|
| Male/Female | 2/18 | 2/18 | N/A |
| Average Age (years) | 46.4 ± 13.22 | 43.9 ± 11.9 | 0.533 |

At the end of the experiment, skin variations in terms of pore, elasticity, wrinkle, texture, and collagen abundance were analyzed and compared for the subjects of the placebo group and of the treatment group. The results are provided in Table 3.

As can be seen in Table 3, in Week 4, the subjects in the treatment group had their skin elasticity obviously better than those in the placebo group. As to variations in skin lines, the placebo group saw an increase of 19.2%, whereas the treatment group had a decrease of 12.8%. Further, the subjects in the treatment group found lines around their eyes largely decreased by about 3.8% (opposite to the 0.1% increase saw in the placebo group). As to collagen abundance, the treatment group had a much larger increase than the placebo group.

The data in Table 3 demonstrate that the *Crassocephalum rabens* extract of the present invention did help increase skin elasticity and collagen, inhibit formation of wrinkles, and improve formed wrinkles. In other words, the *Crassocephalum rabens* extract of the present invention exhibited its potency in improving skin condition.

**Table 3: Skin test results (I)**

| Criterion | Group | Test | Week 0 | Week 4 | P Value |
|---|---|---|---|---|---|
| Pores | Placebo Group | Original Data | 718.4 ± 419.4 | 708.4 ± 394.8 | 0.72^{a} |
| | | Deviation | - | -10.1 ± 127.0 | - |
| | Treatment Group | Original Data | 731.1 ± 371.7 | 696.5 ± 335.6 | 0.18^{a}, 0.92^{b} |
| | | Deviation | - | -34.6 ± 111.1 | 0.52^{b} |
| Elasticity | Placebo Group | Original Data | 50.5 ± 3.0 | 51.3 ± 2.7^{∗∗∗} | < 0.001^{a} |
| | | Deviation | - | 0.8 ± 1.1 | - |
| | Treatment Group | Original Data | 50.3 ± 2.8 | 51.4 ± 3.1 ^{∗∗∗} | < 0.001^{a}, 0.91^{b} |
| | | Deviation | - | 1.1 ± 1.4 | 0.52^{b} |
| Texture | Placebo Group | Original Data | 548.4 ± 402.5 | 567.6 ± 446.8 | 0.38^{a} |
| | | Deviation | - | 19.2 ± 96.5 | - |
| | Treatment Group | Original Data | 434.2 ± 319.8 | 421.4 ± 304.9 | 0.40^{a}, 0.25^{b} |
| | | Deviation | - | -12.8 ± 66.4 | 0.23^{b} |
| Wrinkles | Placebo Group | Original Data | 16.7 ± 13.3 | 16.8 ± 13.8 | 0.94^{a} |
| | | Deviation | - | 0.1 ± 6.2 | - |
| | Treatment Group | Original Data | 12.7 ± 9.6 | 9.0 ± 8.4^{∗∗∗} | 0.002^{a}, 0.04^{b} |
| | | Deviation | - | -3.8 ± 4.6^{#} | 0.03^{b} |
| Collagen Level | Placebo Group | Original Data | 58.3 ± 20.8 | 60.2 ± 20.1^{∗} | 0.04^{a} |
| | | Deviation | - | 1.9 ± 3.9 | - |
| | Treatment Group | Original Data | 53.1 ± 23.4 | 58.2 ± 22.8^{∗∗∗} | < 0.001^{a}, 0.78^{b} |
| | | Deviation | - | 5.2 ± 1.5^{###} | 0.001^{b} |

Moreover, the subjects were evaluated for variations of their skin flecks, UV spots, and brown spots. The results are summarized in Table 4.

From Table 4, it is clear that the two groups showed no significant variation in terms of skin fleck and brown spot. However, the treatment group had a difference of UV spots between Week 0 and Week 4 of -12.8%, whereas the placebo group confirmed a 7.5% increase of UV spots at the end of Week 4 as compared to Week 0.

The results shown in Table 4 suggest that the *Crassocephalum rabens* extract of the present invention was able to inhibit UV-incurred skin spots by preventing skin from absorbing ultraviolet rays and to mitigate UV damages to skin.

**Table 4: Skin test results (II)**

| Criterion | Group | Test | Week 0 | Week 4 | P Value |
|---|---|---|---|---|---|
| Flecks | Placebo Group | Original Data | 120.6 ± 48.4 | 120.8 ± 51.2 | 0.96^{a} |
| | | Deviation | - | 0.2 ± 14.9 | - |
| | Treatment Group | Original Data | 101.6 ± 30.9 | 97.5 ± 28.2 | 0.21a, 0.08^{b} |
| | | Deviation | - | -4.2 ± 14.3 | 0.36^{b} |
| UV Spots | Placebo Group | Original Data | 349.9 ± 56.9 | 357.4 ± 58.3 | 0.15^{a} |
| | | Deviation | - | 7.5 ± 22.3 | - |
| | Treatment Group | Original Data | 347.7 ± 51.2 | 335.5 ± 50.6 | 0.08^{a}, 0.21^{b} |
| | | Deviation | - | -12.2 ± 29.2^{#} | 0.02^{a} |
| Brown Spots | Placebo Group | Original Data | 320.5 ± 101.7 | 320.5 ± 100.5 | 1.00^{a} |
| | | Deviation | - | 0 ± 17.0 | - |
| | Treatment Group | Original Data | 321.1 ± 78.4 | 311.9 ± 77.9 | 0.06^{a}, 0.76^{b} |
| | | Deviation | - | -9.2 ± 20.6 | 0.13^{b} |

Further, the subjects in both the placebo group and the treatment group were evaluated for skin brightness, skin moisture retention, and red areas. The results are shown in Table 5. Therein, a red area is an area where skin had redness and/or irritation.

As reflected in Table 5, the Crassocephalum rabens extract of the present invention significantly improved skin brightness and skin hydration in the subjects within four weeks after the star of administration. This means that the Crassocephalum rabens extract of the present invention effectively increased skin glow and prevented skin defects caused by hydropenia.

**Table 5: Skin test results (III)**

| Criterion | Group | Test | Week 0 | Week 4 | P Value |
|---|---|---|---|---|---|
| Skin Brightness | Placebo Group | Original Data | 57.9 ± 3.1 | 58.0 ± 3.1^{∗∗∗} | < 0.001^{a} |
| | | Deviation | - | 0.2 ± 0.1 | - |
| | Treatment Group | Original Data | 58.3 ± 2.6 | 59.5 ± 2.7^{∗∗∗} | < 0.001^{a}, 0.12^{b} |
| | | Deviation | - | 1.2 ± 1.0^{###} | < 0.001^{b} |
| Skin Moisture Retention | Placebo Group | Original Data | 40.4 ± 8.8 | 40.7 ± 8.1 | 0.29^{a} |
| | | Deviation | - | 0.4 ± 1.4 | - |
| | Treatment Group | Original Data | 39.8 ± 8.5 | 40.9 ± 8.4^{∗∗∗} | 0.02^{a}, 0.95^{b} |
| | | Deviation | - | 1.1 ± 1.8 | 0.18^{b} |
| Red Areas | Placebo Group | Original Data | 190.1 ± 78.2 | 183.1 ± 74.9 | 189.2 ± 92.2 |
| | | Deviation | - | -7.0 ± 25.1 | -0.9 ± 27.3 |
| | Treatment Group | Measurement | 157.9 ± 64.6 | 146.3 ± 59.6 | 153.5 ± 61.0 |
| | | Δ | - | -11.6 ± 18.1 | -4.4 ± 28.3 |

### Example 6: In vitro tests

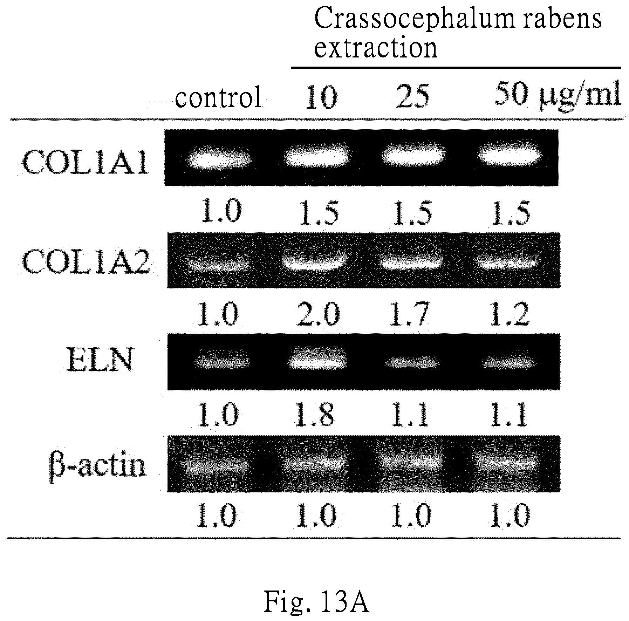

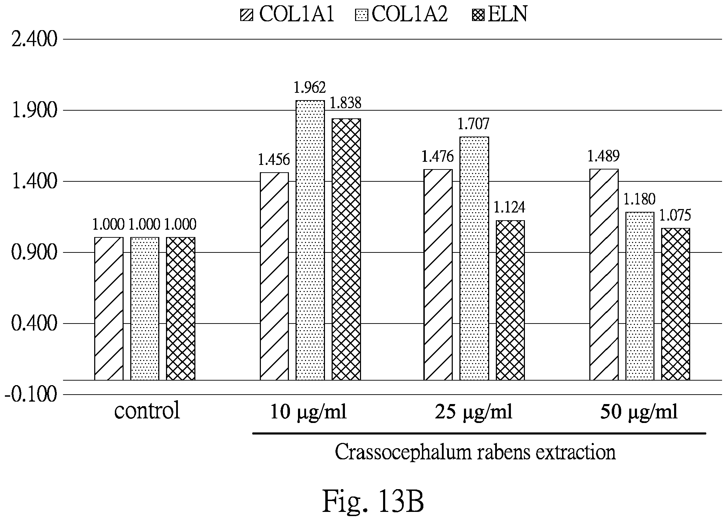

Four groups of identical 3T3 fibroblasts were treated with 0, 10, 25, 50µg/ml of the *Crassocephalum rabens* extract, respectively, for 48 hours and then were measured for expression of COL1A1, COL1A2, and ELN by means of real-time polymerase chain reaction. The results are shown in FIG. 13A and FIG. 13B. Therein, the used *Crassocephalum rabens* extract contained about 7.9% dLGG (1,2-di-O-linolenoyl-3-O-β-galactopyranosyl-sn-glycerol), and every well had a volume of 5ml, so every well was provided with 50-250µg of the *Crassocephalum rabens* extract.

As the data of FIG. 13A and FIG. 13B indicate, the cells treated by the *Crassocephalum rabens* extract of the present invention had more expression of COL1A1, COL1A2, and ELN than their counterparts not treated by the *Crassocephalum rabens* extract of the present invention. The treated cells in all groups had similar COL1A1 expression, whereas the group treated by 10µg/ml of the *Crassocephalum rabens* extract performed better than the other two groups in expression of COL1A2 and ELN.

As people of ordinary skill in the art will appreciate, expression of COL1A1 and COL1A2 is associated with synthesis of collagen in skin cells, while expression of ELN expression of synthesis of elastin. As such, increased expression of COL1A1, COL1A2, and ELN can lead to elevate levels of collagen and elastin in skin cells. Thus, as demonstrated by the result of the example, the *Crassocephalum rabens* extract of the present invention exhibited the activity in enhancing expression of genes critical for the skin structure, such as COL1A1, COL1A2, and ELN, thereby maintaining skin glow and elasticity and smoothening formed wrinkles.

Furthermore, on the basis that a typical composition for external use as designed by the present invention would contain about 0.6% of dLGG, the results of Example 6 suggest suitable dosage of the *Crassocephalum rabens* extract for external use that is about at least 0.66 mg/day and preferably ranges between 0.66 and 3.3 mg/day.

As demonstrated by the results of the examples described above, the *Crassocephalum rabens* extract of the present invention did not incur any adverse reaction in the subjects, and, in virtue of its features of protecting skin against hyaluronidase, inhibiting formation of inflammatory factors and ROS, and promoting anti-oxidation, effectively improved skin hydration, inhibited degradation of collagen and elastin, thereby enhancing skin elasticity, smoothening wrinkles, making sensitive skin healthy, improving skin brightness, and reducing spots. In other words, the *Crassocephalum rabens* extract of the present invention and dLGG, its active ingredient, are believed to have anti-aging effects.

## Claims

1. Use of a composition containing a *Crassocephalum rabens* extract in improving skin texture or in caring skin.

2. The use of claim 1, wherein the *Crassocephalum rabens* extract is rich in dLGG (1,2-di-O-linolenoyl-3-O-β-galactopyranosyl-sn-glycerol).

3. The use of claim 1, wherein the composition containing the Crassocephalum rabens extract is a nutritional supplement.

4. The use of claim 1, wherein the composition containing the *Crassocephalum rabens* extract is a food.

5. The use of claim 1, wherein the composition containing the *Crassocephalum rabens* extract is a skincare product for external use.

6. The use of claim 1, wherein the composition containing the *Crassocephalum rabens* extract is used to decrease skin pores in number and/or shrink skin pores in size.

7. The use of claim 1, wherein the composition containing the *Crassocephalum rabens* extract is used to lighten formed skin spots.

8. Use of a *Crassocephalum rabens* extract in preparing an anti-wrinkle composition.

9. The use of claim 8, wherein the Crassocephalum rabens extract is used to increase a content of collagen in skin cells.

10. The use of claim 8, wherein the *Crassocephalum rabens* extract is used to increase a content of elastin in skin cells.
